# EUROPEAN PATENT APPLICATION

(11) **EP 3 524 290 A1**
(43) Date of publication of application: **14.08.2019**
(21) Application number: 18155947.7
(22) Date of filing: 09.02.2018
(51) Int. Cl.: A61M 1/36, A61K 31/28, A61K 31/69

(54) **METHOD AND SYSTEM FOR MONITORING CARBON MONOXIDE (CO) ADMINISTRATION TO EX-VIVO FLUIDS**

(71) Applicant: Julius-Maximilians-Universitaet Wuerzburg, 97070 Wuerzburg (DE); Albert-Ludwigs-Universität Freiburg, 79085 Freiburg (DE)
(72) Inventor: WOLLBORN, Jakob, 79111 Freiburg (DE); GOEBEL, Ulrich, 79114 Freiburg (DE); SCHICK, Martin, 79285 Ebringen (DE); HERRMANN, Cornellus, 97072 Wuerzburg (DE); WUNDER, Christian, 70192 Stuttgart (DE); MEINEL, Lorenz, 97082 Wuerzburg (DE); STEIGER, Christoph, 97074 Wuerzburg (DE); MERGET, Benjamin, 69198 Schriesheim (DE)
(74) Representative: Teipel, Stephan

(57) **Abstract**

The present invention relates to a method for combined administration of carbon monoxide (CO) to an *ex-vivo* fluid and monitoring of the carbon monoxide administration, said method comprising: (i) generating CO by reacting a CO releasing molecule (CORM) with a release triggering molecule; (ii) administering CO to an *ex-vivo* fluid by contacting the *ex-vivo* fluid with the CO generated in step (i) via a gas-permeable membrane; (iii) analyzing carbon monoxide and/or a carbon monoxide marker after administering in step (ii) CO to the *ex-vivo* fluid by complementary monitoring techniques; (iv) adjusting the CO administration based on the analysis of the carbon monoxide or the carbon monoxide marker carried out in step (iii), if necessary. It furthermore relates to an extracorporeal circuit system for use in the method of the invention.

## Description

### Technical Field

The present invention relates to a method for monitoring carbon monoxide (CO) administration to *ex-vivo* fluids and a system for use in this method. Examples of such ex-*vivo* fluids include blood of an animal or human subject, or perfusion fluids.

### Background

Besides deleterious effects of CO (i.e. poisoning), evidence points towards therapeutic properties of CO in e.g. ischemia-reperfusion-injury and inflammation when used in low concentrations (Motterlini R., Otterbein L.E. (2010), "The therapeutic potential of carbon monoxide", Nat. Rev. Drug Discov., doi: 10.1038/nrd322).

Extracorporeal therapy is used to treat acute or chronical ill patients as well as in a perioperative setting: In cardiothoracic surgery, cardiopulmonary bypass (CPB), consisting of an oxygenator, a pump, tubing and cannulas is employed to replace cardiocirculatory and pulmonary function during non-heart-beating surgery. Diseases accompanying acute or chronic cardiac, respiratory, renal or hepatic failure are frequently treated with extracorporeal therapy. Furthermore, organs can be perfused using extracorporeal circuits and thus be preserved for transplantation. All of the above mentioned disorders and procedures are often based on or followed by severe inflammatory reactions or ischemia-reperfusion-injury. Particularly, in cardiopulmonary support (e.g. after myocardial infarction, cardiac arrest, acute respiratory distress syndrome, etc.), extracorporeal membrane oxygenation (ECMO) or extracorporeal life support (ECLS) are employed. Therefore, large blood vessels (veins and/or arteries) are cannulated and a circuit is set-up using an oxygenator, a pump and tubing. Thus, short- or even sometimes long-term duration of organ assist or replacement is feasible in a critical care setting.

US 2007/0202083 A1 relates to a method for transplanting an organ, wherein the recipient of the organ is administered with a pharmaceutical composition comprising carbon monoxide in an amount sufficient to enhance survival of the transplanted organ in the recipient. In order to form the carbon monoxide composition, carbon monoxide gas is bubbled directly into a liquid until the desired concentration of carbon monoxide in the liquid is reached. As an alternative, an appropriate liquid is passed through tubing that allows gas diffusion, where the tubing runs through an atmosphere comprising carbon monoxide, e.g. using an extracorporeal membrane oxygenator. The carbon monoxide diffuses into the liquid to create a liquid carbon monoxide composition. Said liquid is typically an aqueous solution which can be orally delivered to a patient or by injection. However, said method described in US 2007/0202083 A1 has the disadvantage that CO concentration in the liquid is difficult to control

Further modes of CO application include inhalation and oral or systemic administration as "carbon monoxide releasing molecules" (CORM). However, these methods also contain some risks: inhalation of CO implies providing a reservoir (e.g. a gas cartridge) of the odorless and dangerous gas at the site of utilization. Not only does this include a risk to harm patients with fatal overdose (small dosing errors can cause considerable harm), but it also presents a source of danger for staff involved. Thus, uncontrolled application is always associated with a risk of intoxication, which may cause lethal threats. Of note, these problems can be avoided by using CORMs by releasing certain amounts of CO in a type-specific controlled fashion. CORMs are typically metal carbonyl complexes (e.g. molybdenum, ruthenium) which release CO in response to a physiological trigger (e.g. sulfite species, water, etc.). Thus, CO release from these CORMs can be tailored with reasonably quantitative and time-dependent control by using medicinal chemistry principles.

Despite these favorable properties, CORMs exhibit substantial disadvantages. Typically, CORMs degrade into undefined and potentially harmful degradation products (various oxidation states, ligand states, conformity, etc.), thereby significantly challenging any medical-translational approach. This issue was recently addressed by introducing a membrane-based Therapeutic Gas Releasing System (TGRS), enabling clinically significant carbon monoxide release while preventing patient exposure with potentially toxic degradation products (EP 15150532.8 and DE 10 2014 008 685.2; Steiger C. et al. (2015) "Controlled therapeutic gas delivery systems for quality-improved transplants", Eur. J. Biopharm, doi: 10.106/j.ejpb.2015.10.009). Within TGRS, a hydrophobic membrane (silicone) separates the patient or the addressed tissue from a reaction chamber, in which CO release from a CORM is induced by variable trigger (e.g. water, light etc.). After CO release has been initiated, solely the gas can permeate through the membrane, thereby leaving the reaction chamber, and triggering therapeutic effects at the patient/tissue. Potentially harmful degradation products of the CORM are safely retained within the release system.

Despite the above, there is still a need for methods and systems that allow the administration of CO to *ex-vivo* fluids in a highly controllable and safe manner, wherein both, transition metal toxicity and CO dosing challenges are addressed, in order to enable their use in clinical scenarios.

### Brief Description of the Invention

This problem is solved by
(1) A method for combined administration of carbon monoxide (CO) to an *ex-vivo* fluid and monitoring of the carbon monoxide administration, said method comprising:
   (i) generating CO by reacting a CO releasing molecule (CORM) with a release triggering molecule,
   (ii) administering CO to an *ex-vivo* fluid by contacting the *ex-vivo* fluid with the CO generated in step (i) via a gas-permeable membrane,
   (iii) analyzing carbon monoxide and/or a carbon monoxide marker after administering in step (ii) CO to the *ex-vivo* fluid by complementary monitoring techniques,
   (iv) adjusting the CO administration based on the analysis of the carbon monoxide or the carbon monoxide marker carried out in step (iii), if necessary.

Preferred embodiments of the method of the invention relate to:
(2) The method according to item (1), wherein the *ex-vivo* fluid is blood of a subject or perfusion liquid, and preferably blood of a subject.
(3) The method according to item (1) or (2), wherein the carbon monoxide marker is carboxyhemoglobin (COHb).
(4) The method according to any of items (1) to (3), wherein the complementary monitoring techniques include at least two of: COHb measurement in a blood sample of a subject, measuring the CO concentration in the exhaled breath of a subject, and measuring the CO concentration in the exhaust air of an oxygenator, the oxygenator being integrated in a cardiopulmonary support system connected with the blood cycle of a subject.
(5) The method according to any of items (1) to (4), wherein the complementary monitoring techniques applied in step (iii) is supported by machine learning-based CO level prediction, preferably by using Random Forest regression.

This object is further solved by:
(6) An extracorporeal circuit system for use in a method of the invention, said extracorporeal circuit system comprising:
   an extracorporeal carbon monoxide releasing system (ECCORS) comprising a membrane module with an outer and an inner compartment,
   wherein the outer compartment is connected to a primary circuit carrying blood of a subject, and the inner compartment is connected to a secondary circuit wherein CO is generated by reacting a CO releasing molecule with a release triggering molecule; and wherein the two compartments in the membrane module are separated from each other by a gas-permeable membrane that allows CO permeation generated in the secondary circuit from the inner compartment into the outer compartment, thereby administering CO to the *ex-vivo* fluid,
wherein the primary circuit is connected with the blood cycle of a subject.

Preferred embodiments of the system of the present invention are directed to:
(7) The extracorporeal circuit system according to item (6), wherein the inner and the outer compartment of the membrane module are separated by tube membranes.
(8) The extracorporeal circuit system according to item (6) or (7), wherein the primary circuit further comprises an oxygenator and a pump.

Preferred embodiments of the method and the system of the present invention are directed to:
(9) The method according to any of items (1) to (5) and the extracorporeal circuit system for use according to any of items (6) to (8), wherein the gas-permeable membrane is a silicone membrane or a PTFE (polytetrafluoroethylene) membrane.
(10) The method according to any of items (1) to (5) and (9) and the extracorporeal circuit system for use according to any of items (6) to (9), wherein the CORM is a metal carbonyl compound.
(11) The method according to any of items (1) to (5), (9) and (10) and the extracorporeal circuit system for use according to any of items (6) to (10), wherein the CORM is a metal carbonyl compound selected from the group consisting of Mo(CO)₃(CNCH₂COOH)₃ ("Beck1"), Mo(CO)₃(CNCH₂COONa)₃ ("Beck1-Na"), CORM-ALF794, CORM-1, CORM-2, CORM-3, and CORM-401.
(12) The method according to any of items (1) to (5) and (9) to (11) and the extracorporeal circuit system for use according to any of items (6) to (11), wherein the release triggering molecule is selected from the group consisting of a sulfur containing compound, a nitrogen containing compound, and an oxidizing compound.
(13) The method according to any of items (1) to (5) and (9) to (12) and the extracorporeal circuit system for use according to any of items (6) to (12), wherein the release triggering molecule is an oxidizing compound selected from the group consisting of iron(III)chloride (FeCl₃), potassium permanganate (KMnO₄), cer(IV)sulfate (Ce(SO₄)₂), potassium dichromate K₂Cr₂O₇, gold(III)chloride (AuCl₃), and silver nitrate (AgNO₃).
(14) The method according to any of items (1) to (5) and (9) to (13) and the extracorporeal circuit system for use according to any of items (6) to (13), wherein the CORM is added to an aqueous solution of the release triggering molecule or the release triggering molecule is added to an aqueous solution of the CORM.
(15) The extracorporeal circuit system for use according to items (5) to (14), wherein the extracorporeal circuit system is part of a cardiopulmonary support system or a dialysis system.

The present invention will be explained in detail in the following detailed description and illustrated in Examples 1 and 2.

### Detailed description of the invention

An *"ex-vivo* fluid" in the context of the present invention means a fluid that is administered with carbon monoxide outside the body of a subject.

The *ex-vivo* fluid can e.g. be blood from a subject, which, after being administered with CO outside the body of the subject (i.e. extracorporeally) by means of the method of the present invention is circulated back into the blood cycle of a subject, using the system of the present invention. A "subject" in the context of the present invention means an animal, in particular a mammal such as a monkey, rat, mouse, dog, cat, pig, cow or horse, or a human patient.

The *ex-vivo* fluid can also be a perfusion liquid for administration to a subject, e.g. during surgery. Suitable perfusion liquids for such purposes are known to the skilled person. A suitable perfusion liquid for use in the present invention is for example isotonic saline solution.

In the context of the method and the system of the present invention, blood of a subject is preferred as the *ex-vivo* fluid.

A "carbon monoxide releasing molecule" ("CORM"), as used in the present invention, means a compound that releases carbon monoxide (CO) upon reaction with a CO release triggering compound. Carbon monoxide releasing compounds which are useful for the methods and systems of the present invention will further be described herein below.

A "release triggering molecule" (or "triggering molecule" or "trigger molecule" or "triggering compound" or "trigger compound"), as used in the present invention, means a compound that reacts with the carbon monoxide releasing molecule in a way that carbon monoxide is released from the carbon monoxide releasing molecule. Triggering molecules which are useful for the methods and systems of the present invention will further be described herein below.

A "CO-containing solution", as used herein, is a solution, typically an aqueous solution that contains carbon monoxide gas. Said carbon monoxide gas is generated by reacting a carbon monoxide releasing molecule with a release triggering molecule. The CO-containing solution thus also contains a CORM and a release triggering compound, at least as long the CO-releasing reaction between the CORM and the release triggering compound has not been finished.

"Extracorporeal", as sued herein, means outside the body. Of course, this terminology does not exclude that the extracorporeal circuit system of the present invention is connected via cannulas with the blood stream of a subject.

The "extracorporeal carbon monoxide releasing system" ("ECCORS") used in the present invention enables CO administration to an *ex-vivo* fluid, preferably to the blood of a subject, via a gas-permeable, and liquid- and solid-impermeable membrane from a CO-containing solution in the method and system of the present invention.

For this purpose, the extracorporeal carbon monoxide releasing system comprises a membrane module, which is preferably a silicone membrane module, as a core functional element of the ECCORS. Tube membranes, which are preferably silicone tube membranes, separate an outer compartment and an inner compartment. The outer compartment typically carries the *ex-vivo* fluid, i.e. blood of a subject or a perfusion liquid, preferably the blood of a subject. The inner compartment typically carries a solution wherein CO is generated by reacting a CORM with a release triggering compound. Preferably, a plurality of tube membranes (up to 10.000, preferably up to 5000, more preferably up to 2000 tube membranes) is used in order to separate the outer compartment and the inner compartment in the membrane module. The tube membranes used in the membrane module are thus preferably hollow fibers.

With the ECCORS being integrated in the extracorporeal circuit system of the invention, the outer compartment of the membrane module is connected to a primary circuit which is connected with the blood cycle of a subject, and the inner compartment of the membrane module is connected to a secondary circuit wherein a solution of the CO release triggering molecule or of the CORM is circulated. In order to generate CO in the secondary circuit, the CORM is added to the solution of the release triggering molecule, or the release triggering molecule is added to the solution of the CORM. The secondary circuit is thus part of both the extracorporeal carbon monoxide releasing system" ("ECCORS") and the extracorporeal circuit system of the invention.

The "extracorporeal circuit system" as described herein enables administration of CO to the blood of a subject outside of the body of the subject, recirculation of the blood into the body of a subject, and monitoring CO administration to the *ex-vivo* fluid by applying the method according to the present invention.

In the primary circuit of the extracorporeal circuit system, the blood is circulated in tubes. Circulation is achieved by means of a pump that is part of the primary circuit of the extracorporeal circuit system of the invention.

In the system of the present invention, the blood of a subject, can be recirculated into the body of the subject after it has been administered with CO outside the body using the method and the system of the present invention. The recirculation of the CO enriched blood of the subject, can be achieved using cannulas for connecting the primary circuit of the extracorporeal circuit system of the invention to the blood vessels (veins and/or arteries) of the subject. The monitoring (analysis) step included in the method of the present invention allows control of CO administration and avoids overdosing.

The primary circuit of the extracorporeal circuit system of the present invention preferably further includes an oxygenator for enriching the blood of a subject with oxygen and removing CO₂ therefrom outside the body. This is necessary in order to replace or to support pulmonary function of a subject when external life support is required (e.g. during heart surgery).

In the secondary circuit of the system of the present invention, CO is typically generated in an aqueous solution. This can either be accomplished by adding a CORM to an aqueous solution of a CO release triggering compound, or by adding a release triggering compound to an aqueous solution of a CORM. Of course, the compound that is added to an aqueous solution of the other compound can also be in aqueous solution.

The resulting CO-containing solution is circulated in the secondary circuit using a pump, preferably a tube pump. Preferably, the secondary circuit also comprises at least one gastight bag, preferably at least two gastight bags for preventing overpressure in the system. CO release can for example be controlled if small portions of the CORM are added to the aqueous solution of the release triggering compound. For this purpose, the secondary circuit carrying the aqueous solution of the release triggering compound typically comprises a port for CORM injection. This allows addition of small amounts of CORM and thereby a controlled release of carbon monoxide into the system of the present invention.

In the extracorporeal circuit system of the present invention, a separation of two circuits is accomplished, which allows generation of CO independently from the blood flow of a subject in a controlled manner. By dividing the extracorporeal circuit system of the invention into two circuits which are separated by the gas-permeable, but otherwise impervious membrane in the membrane module of the extracorporeal carbon monoxide releasing system (ECCORS), it is possible to protect organs from the ischemia-reperfusion-injury after recirculation of the blood of the subject enriched outside the body of the subject with CO into the blood stream of the subject.

For generating CO in the method and in the system according to the present invention, a reaction between a CORM and a release triggering compound is required.

The CO releasing molecule (CORM) used in the method and the system according to the present invention is preferably a metal carbonyl compound. The metal carbonyl compound comprises e.g. a complex of an element of the group of Rh, Ti, Os, Cr, Mn, Fe, Co, Mo, Ru, W, Re, and Ir. More preferably, the metal carbonyl compound comprises a complex of an element of the group of Rh, Mo, Mn, Fe, and Ru, even more preferably of the group of Rh, Fe, Mn, and Mo. The metal carbonyl compounds may be regarded as complexes, because they comprise CO groups coordinated to a metal center. However, the metal may be bonded to other groups by other than coordination bonds, e.g. by ionic or covalent bonds. Thus, groups other than CO, which form part of the metal carbonyl compound, need not strictly be "ligands" in the sense of being coordinated to a metal center via a lone electron pair, but are referred to herein as "ligands" for ease of reference.

Thus, the ligands to the metal may all be carbonyl ligands. Alternatively, the carbonyl compound may comprise at least one ligand which is not CO. Ligands which are not CO are typically neutral or anionic ligands, such as halide, or derived from Lewis bases and having N, P, O or S or a conjugated carbon group as the coordinating atom(s). Preferred coordinating atoms are N, O and S. Examples include, but are not limited to, sulfoxides such as dimethylsulfoxide, natural and synthetic amino acids and their salts for example, glycine, cysteine, and proline, amines such as NEt₃ and H₂NCH₂CH₂NH₂, aromatic bases and their analogues, for example, bis-2,2'-pyridyl, indoline, pyrimidine and cytidine, pyrroles such as biliverdin and bilirubin, drug molecules such as YC-1(2-(5'-hydroxymethyl-2'-furyl)-1-benzylindazole), thiols and thiolates such as EtSH and PhSH, chloride, bromide and iodide, carboxylates such as formate, acetate, and oxalate, ethers such as Et₂O and tetrahydrofuran, alcohols such as EtOH, and nitriles such as MeCN. Other possible ligands are conjugated carbon groups, such as dienes, e.g. cyclopentadiene (C₅H₅) or substituted cyclopentadiene. The substituent group in substituted cyclopentadiene may be for example an alkanol, an ether or an ester, e.g. -(CH₂)ₙOH wherein n is 1 to 4, particularly -CH₂OH, - (CH₂)ₙOR wherein n is 1 to 4 and R is hydrocarbon, preferably alkyl of 1 to 4 carbon atoms and -(CH₂)ₙOOCR wherein n is 1 to 4 and R is hydrocarbon preferably alkyl of 1 to 4 carbon atoms. The preferred metal in such a cyclopentadiene or substituted cyclopentadiene carbonyl complex is Fe.

Preferably, Mo(CO)₃(CNCH₂COOH)₃ ("Beck1"), Mo(CO)₃(CNCH₂CONaO)₃ ("Beck1-Na") Mo(CO)₃(CNC(CH₃)₂COOH)₃, CORM-ALF794, CORM-1, CORM-2, CORM-3, or CORM-401 is used as carbon monoxide releasing molecule in the methods and systems according to the present invention. Particularly preferred for use in the present invention is Mo(CO)₃(CNCH₂COOH)₃ ("Beck1 ") The compound "Beck1" is disclosed in D. Achatz et al., "Carbonyl-Komplexe von Chrom, Molybdän und Wolfram in Isocyanacetat. Reaktionen am koordinierten Isocyanacetat. Stabilisierung von Isocyanessigsäure und Isocyanacetylchlorid am Metall. Isocyanopeptide [1]", Zeitschrift für anorganische und allgemeine Chemie, 631 (2005), 2339-2346. The other compounds are disclosed in WO 2016/110517 A1.

It is also explicitly referred to WO 2008/130261 A1 and US 2007/0219120 A1 for a description of CO releasing molecules that can also, but not preferably be used in the methods and systems of the present invention. There, aldehydes according to formula I 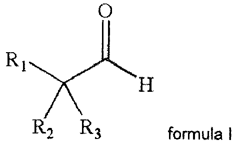 are disclosed which can also be used as gas releasing molecules in the methods and systems of the present invention, wherein R₁, R₂ and R₃ are each independently selected from alkyl, substituted alkyl, cycloalkyl, substituted cycloalkyl, heterocyclyl, substituted heterocyclyl, alkylheterocyclyl, substituted alkylheterocyclyl, alkenyl, substituted alkenyl, aryl, substituted aryl, heteroaryl, substituted heteroaryl, alkylaryl, substituted alkylaryl, wherein the number of C atoms is 1-12 or 1-6 in each case hydroxyl, alkoxy, amino, alkylamino, mercapto, alkylmercapto, aryloxy, substituted aryloxy, heteroaryloxy, substituted heteroaryloxy, alkoxycarbonyl, acyl, acyloxy, acylamino, alkylsufonyl, alkylsulfinyl, F, CI, Br, NO₂ and cyano; or two or more of R₁, R₂ and R₃ are taken together to form a substituted or unsubstituted carbocyclic or heterocyclic ring structure or a derivative thereof. For any substituent the number of C atoms is 1-12 or 1-6.

A derivative of a compound of formula I being an acetal, hemiacetal, aminocarbinol, aminal, imine, enaminone, imidate, amidine, iminium salt, sodium bissulfite adduct, hemimercaptal, dithioacetal, 1,3-dioxepane, 1,3-dioxane, 1,3-dioxalane, 1,3-dioxetane, α-hydroxy-1,3-dioxepane, α-hydroxy-1,3-dioxane, α-hydroxy-1,3-dioxalane, α-keto-1,3-dioxepane, α-keto-1,3-dioxane, α-keto-1,3-dioxalane, α-keto-1,3-dioxetane, macrocylic ester/imine, macrocyclic ester/hemiacetal, oxazolidine, tetrahydro-1,3-oxazine, oxazolidinone, tetrahydro-oxazinone, 1,3,4-oxadiazine, thiazolidine, tetrahydro-1,3-thiazine, thiazilidinone, tetrahydro-1,3-thiazinone, imidazolidine, hexahydro-1,3-pyrimidine, imidazolidinone, tetrahydro-1,3-pyrimidinone, oxime, hydrazine, carbazone, thiocarbazone, semicarbazone, semithiocarbazone, acyloxyalkyl ester derivative, O-acyloxyalkyl derivative, N-acyloxyalkyl derivative, N-Mannich base derivative or N-hydroxymethyl derivative can also be used as gas releasing molecules in the present invention.

The gas releasing molecule in the present invention can e.g. also be trimethylacetaldehyde, 2,2-dimethyl-4-pentenal, 4-ethyl-4-formyl-hexanenitrile, 3-hydroxy-2,2-dimethylpropanal, 2-formyl-2-methyl-propylmethanoate, 2-ethyl-2-methylpropionaldehyde, 2,2-dimethyl-3-(p-methylphenyl)propanal or 2-methyl-2-phenylpropionaldehyde.

Oxalates, oxalate esters or amides can also, but not preferably be used as carbon monoxide releasing molecules in the methods and systems of the present invention.

Furthermore, but not preferably, carboxyboranes, carboxyborane esters or carboxyborane amides can be used as carbon monoxide releasing molecule in the present invention. Such gas releasing molecules are particularly described in WO 2005/013691 A1. In one embodiment of the present invention

BHₓ(COQ)_{y}Z_{z}

is thus used as gas releasing molecule wherein x is 1, 2 or 3, y is 1, 2 or 3, z is 0, 1 or 2, x + y + z = 4, each Q is O⁻, representing a carboxylate anionic form, or is OH, OR, NH₂, NHR, NR₂, SR or halogen, where the or each R is alkyl (preferably of 1 to 4 carbon atoms), each Z is halogen, NH₂, NHR', NR'₂, SR' or OR' where the or each R' is alkyl (preferably of 1 to 4 carbon atoms). More preferably, z is 1 and/or y is 1 and/or x is 3. In one embodiment, at least one Q is O⁻ or OR and the composition includes at least one metal cation, wherein the metal cation is preferably an alkali metal cation or an earth metal cation.

When a boronocarboxylate is used as gas releasing molecule, it is most preferably Na₂(H₃BCO₂), also known as CORM-A1.

The CO releasing molecule releases CO upon contact with the release triggering compound. "Contact" thus means that a reaction between the gas releasing molecule and the trigger compound can take place which results in carbon monoxide release from the carbon monoxide releasing molecule.

The release triggering compound can e.g. be a sulfur-containing compound or a nitrogen-containing compound, an oxidizing compound, an acid or a base or water.

When the carbon monoxide releasing molecule is a metal carbonyl compound, which is the preferred carbon monoxide releasing molecule in the present invention, the trigger compound is preferably a carbonyl substituting ligand, such as a sulfur-containing compound or a nitrogen-containing compound. The sulfur-containing compound can e.g. be selected from an alkali metal or alkaline-earth metal salt, preferably a sodium salt, of sulfite, dithionite, or metabisulfite, or a compound bearing at least one thiol moiety, such as cysteine or glutathione.

Examples of oxidizing compounds useful as trigger compound in the methods and systems of the present invention, in particular for triggering CO release from the preferred metal carbonyl compounds, include peroxides, perborates, percarbonates, and nitrates of which calciumperoxide, dibenzoylperoxide, hydrogenperoxide urea, sodium perborate, and sodium percarbonate are preferred. Further, oxidizing metal salts, such as iron(III)chloride (FeCl₃), potassium permanganate (KMnO₄), cer(IV)sulfate (Ce(SO₄)₂), potassium dichromate (K₂Cr₂O₇), gold(III)chloride (AuCl₃), and silver nitrate (AgNO₃) can be used for triggering CO release from the carbon monoxide releasing molecule, in particular from the preferred metal carbonyl compounds. Iron(III)chloride, potassium permanganate, and cer(IV)sulfate are preferred as oxidizing metal salts.

As acid, e.g. citric acid can be used. In one embodiment, the trigger compound is a non-enzymatic compound. The trigger compound can also be water or a solvent. A preferred gas releasing molecule which releases carbon monoxide upon contact with water is ALF186.

Not being bound to the theory, but for a metal carbonyl compound as carbon monoxide releasing molecule and a sulfur-containing compound or other electron withdrawing compound as the trigger compound, it is e.g. believed that, when this trigger compound comes into contact with the metal carbonyl compound, a ligand substitution takes place thereby triggering gas release. When the metal carbonyl compound come into contact with an oxidizing triggering compound, it is believed that the oxidation of the metal of the metal carbonyl compound leads to a breakup of the metal carbonyl compound thereby releasing CO.

In the method and system for use in the method of the present invention, the trigger compound is typically provided in an aqueous solution. For example, if an oxidizing metal salt is used as trigger compound, the concentration of the metal salt in the aqueous phase is typically in a range of 0.01 -20 mol/l, preferably in a range of 0.02-15 mol/l, and particularly preferred in a range of from 0.05-10 mol/l. The higher the concentration of the oxidizing salt in the aqueous solution is, the stronger is the oxidizing effect on the carbon monoxide releasing molecule, which increases the rate of CO release. The CORM is then added to the aqueous solution of the triggering compound in order to generate carbon monoxide. Preferably, the CORM is added to the aqueous solution of the triggering compound also in the form of an aqueous solution, wherein the concentration of the CORM, depending on its aqueous solubility, is typically in the range of from 0.01-2 mol/L, preferably in the range of from 0.05-1.5 mol/L, more preferably in the range of from 0.01-1 mol/L.

In a preferred embodiment of the method and system of the present invention, FeCl₃ is provided as the triggering compound and Beck1 as the carbon monoxide releasing molecule. FeCl₃ is then provided in aqueous solution in a concentration of 1-10 mol/L, preferably 1.5-7.5 mol/L, more preferably 2-5 mol/L. Beck1, also provided in aqueous solution in a concentration of 0.01-1 mol/L, preferably 0.05-0.5 mol/L, is then added to the solution of the triggering compound. In an alternative embodiment, Ce(SO₄)₂ can be used (in aqueous solution in the same concentrations as with FeCl₃) as the triggering compound instead of FeCl₃.

In the method and system of the present invention, it is however also possible to add the triggering compound to an aqueous solution of CORM as described above in order to generate carbon monoxide. Typically, the triggering compound is then added in form of an aqueous solution as described above to the aqueous solution of the CORM. In that case, the same concentrations for the aqueous CORM solution and the aqueous release triggering compound as described above can be used.

In step (ii) of the method according the invention, CO generated in step (i) of the method according to the invention is administered to an *ex-vivo* fluid by contacting the *ex-vivo* fluid with the CO via a gas-permeable membrane. Typically, the CO is generated in aqueous solution, as described above, by reaction of a CORM with a release triggering compound.

The "gas permeable membrane" used in the method and the system according to the present invention is gas permeable and liquid and solid impermeable. It typically comprises one or more of the following materials of the group consisting of polytetrafluoroethylene (PTFE, Teflon), silicone, butyl rubber, cellulose acetate, ceramic, dimethyl silicone rubber 60, ethyl cellulose, fluorosilicone, Kel F, Latex, methyl cellulose, metal organic framework membranes (see e.g. Chem. Soc. Rev., 2014, 43, 6116-6140), mylar, natural rubber, nitrile silicone, nylon, polycarbonate, polyethersulfone, polyethylene, polypropylene, polystyrene, polyvinylalcohol, polyvinyl chloride, polyvinylidene fluoride, vinylidene chloride-vinyl chloride and zeolith. Such a membrane is also described in WO 2016/110517 A1. Silicone and polytetrafluoroethylene are the preferred membrane materials with silicone being particularly preferred.

The gas permeable and liquid and solid impermeable membrane used in the present invention allows that CO, which is typically generated in an aqueous solution from a carbon monoxide releasing molecule that is reacted with a triggering molecule, can be administered to an *ex-vivo* fluid, such as blood or perfusion liquid, without solid or dissolved residues of the CORM or the triggering molecule coming into contact with the *ex-vivo* fluid. Potentially toxic solid residues from the reaction of the CORM with the triggering molecule are thus maintained in the solution wherein the CO is generated, and do not contaminate the *ex-vivo* fluid. Ultimately, this e.g. allows the recirculation of blood into the blood cycle in the body of a subject, and the transplantation of an *ex-vivo* organ stored in a perfusion liquid into the body of a subject, respectively.

Both procedures, i.e. adding the CORM to the triggering compound or vice versa allow controlled CO generation by tailorable injection kinetics of the CORM and the triggering compound, respectively, and, in consequence, controlled administration of CO to blood or an perfusion liquid via the gas-permeable membrane. Therapeutic COHb levels in the blood are in the range of 10±4%, based on the total blood-hemoglobin. Suitable CO levels in a perfusion liquid for storing organs and tissue before transplantation are in the range of 20-200 µM.

The method of the present invention requires in step (iii) analyzing carbon monoxide and/or a carbon monoxide marker after administering in step (ii) CO to the *ex-vivo* fluid by complementary monitoring techniques. This step of the claimed method allows permanent assessment of systemic CO levels of a subject, and, accordingly, controlled administration of CO to an *ex-vivo* fluid, in particular to the blood of a subject, which can subsequently be (re)circulated into the body of the subject. A carbon monoxide marker is for example COHb (carboxyhemoglobin).

The complementary monitoring methods for analyzing CO application in step (iii) of the method of the present invention include:
(a) COHb measurement using blood gas analysis: After a subject has been administered with blood or a perfusion liquid administered with CO outside the body of the subject using the method and/or the system of the present invention, COHb as a CO marker can be determined in a blood sample the subject (typically in a blood sample of 0.1 - 0.5 mL taken from the patient or from the primary circuit of the circuit system of the invention) of the extracorporeal blood administered with CO by the method according to the present invention. Typically, a COHb value of not more than 10±3%, based on the total blood-hemoglobin, is attempted. COHb measurement using blood gas analysis is illustrated in the example of the present description (see also Roth, D., et al., Ann. Emerg. Med. 2011, 58(1), 74-79)
(b) CO concentration in exhaled breath of a subject: After a subject has been administered with blood or a perfusion liquid administered with CO outside the body of the subject using the method and/or the system of the present invention, the CO level of the subject can be calculated on the basis of the exhaled air from the subject. Typically, a CO threshold concentration of not more than 250 ppm CO in the expired air of the subject is attempted. Determining the CO concentration in exhaled breath is illustrated in the example of the present description.
(c) CO concentration in the exhaust air of the oxygenator: After a subject has been administered with blood or a perfusion liquid administered with CO outside the body of the subject using the method and/or the system of the present invention, the CO level of the subject can be quantified on the basis of the oxygenator's exhaust air, with the oxygenator forming part of the system of the present invention. Typically, a CO threshold concentration of not more than 100 ppm CO in the oxygenator air is attempted. Determining the CO concentration in exhaled breath of a subject is illustrated in the example of the present description.
(d) Pulse oximetric analysis of COHb: After a subject has been administered with blood or a perfusion liquid administered with CO outside the body of the subject using the method and/or the system of the present invention, the CO level of the subject can be quantified on the basis, the CO level of the subject can be quantified using pulse oximetric analysis of COHb. However, quantification of COHb with pulse oximetry suffers from a low sensitivity as low as 48% [M. Touger et al. (2010) Performance of the RAD-57 pulse CO-oximeter compared with standard laboratory COHb measurement. Ann. Emerg. Med. 56:382-388. Doi: 10.106/j.annemergmed.2010.03.041], and therefore represents a less adequate method for reliably monitoring the level of CO following administration of CO to the blood of a subject. In the example of the present description, the insufficient relationship of COHb measurement by pulse oximetry and blood gas analysis is illustrated. Pulse oximetric analysis of COHb is therefore preferably not used as the only method for monitoring the level of CO following administration of CO to the blood of a subject in the present invention, but preferably in combination with at least one of methods (a) to (c).

The system of the present invention comprises the respective analytical means for carrying out the above described analytical methods according to (a) to (d).

Combining the above analytical procedures (a) to (d) with the purpose of reliably monitoring and controlling the delivered quantity of CO by the extracorporeal carbon monoxide releasing system is desirable in the method of the present invention. Complementary methods means that at least two of: COHb measurement in a blood sample of a subject (according to (a) and/or (d)), measuring the CO concentration in the exhaled breath of a subject (according to (b)), and measuring the CO concentration in the exhaust air of an oxygenator (according to (c)) are used for analyzing CO and/or a carbon monoxide marker after CO administration to the *ex-vivo* fluid, in particular to the blood of a subject. This allows assessment of CO levels in the blood of a subject, and the initiation of safety measures in case of over-dosing.

It is preferred to use more than two of the above methods in order to determine the CO content in the *ex-vivo* fluid, in particular the blood of a subject. It is particularly preferred in the method and system of the invention that systemic levels of CO are constantly assessed using all four complementary monitoring techniques (a) to (d). The data obtained from the analysis is then processed in an actual-target comparison approach, controlling the CO release rate and hence systemic CO level within a subject, in particular a human patient whose blood cycle is connected with the system of the present invention. In case of unforeseen overdoses, an emergency bypass of the system can be induced.

The safety and potential of CO delivery using the ECCORS depends highly on analytical precision, trueness, and accuracy of those parameters that are being assessed as monitoring parameters predicting systemic CO levels. In order to further improve the analytical strength of the CO monitoring according to step (iii) of the method of the invention, a machine learning approach has been established. This approach can be applied for processing the real-time CO monitoring parameters to provide a predicted CO level value for subsequent modification of the feedback loop. The random forest based variant of this approach, which is illustrated in Example 2, allows improving the analytical power of the delivery approach thereby significantly increasing the translational significance of the concept.

Based on the analysis of the carbon monoxide and/or the carbon monoxide marker, the carbon monoxide administration to the *ex-vivo* fluid is adjusted in step (iv) of the method according to the invention, if necessary.

This means that if the required CO levels have not yet been reached, CO release is increased by either adding more CORM to the release triggering compound or more of the release triggering compound to the CORM. In particular, this also means that in case of unintentional overdosing, the system according to the present invention, which is used in the method of the present invention, includes a safety mechanism, which allows that the extracorporeal carbon monoxide releasing system (ECCORS) is automatically bypassed in order to stop increase of CO level within the blood while continuously enabling further extracorporeal support. Excess CO can also be washed out from the body by increasing sweep gas flow to the oxygenator. Due to the narrow therapeutic window of CO, these options are crucial for any emergency care application, where CO is used by systemic application in quantities that have been associated with therapeutic effects (i.e. COHb 10±4%). Thus, in case that any of the predetermined thresholds (exemplary: 13 % COHb, 250 ppm CO expired air, 100 ppm oxygenator air) is exceeded, the CO delivery system is bypassed, thereby instantaneously capping CO supply and limiting the systemic CO concentration to nontoxic levels.

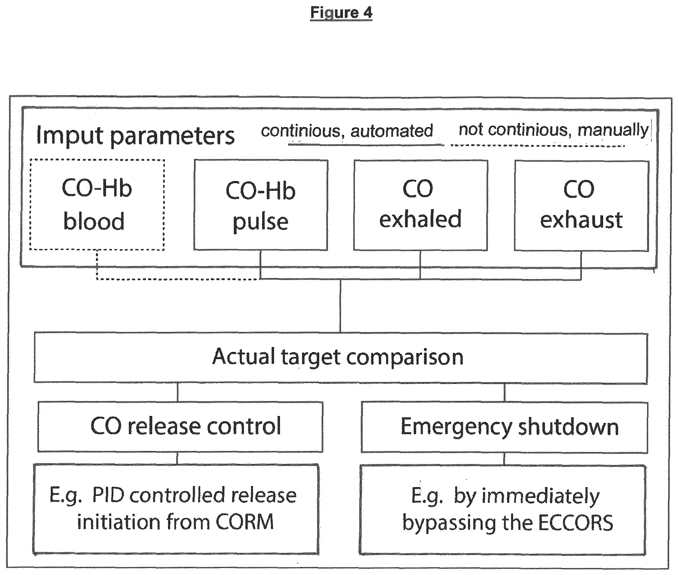

In one embodiment of the method and the system of the present invention, systemic CO delivery from the extracorporeal carbon monoxide releasing system is tailored by controlled injection of release triggering substance. The injection is controlled by a feedback loop comprising a feedback algorithm including but not limited to proportional-integral-derivative (PID) control with (a) COHb measurement using blood gas analysis, (b) CO in exhaled breath, (c) CO in the exhaust air of the oxygenator being input parameters, and (d) pulse oximetric analysis of COHb. Desired COHb levels can hence be automatically maintained within the desired range. In case that all or any of the monitoring parameters exceed a predefined threshold, the extracorporeal carbon monoxide releasing system can be automatically or manually bypassed. **Figure 4** shows an example of an algorithm controlling an extracorporeal carbon monoxide releasing system connected to an extracorporeal circuit system according to the invention.

In conclusion, the present invention provides a method and a system allowing controlled release and administration of CO to an *ex-vivo* fluid, in particular blood of a subject, outside the body of a subject, wherein high and potentially toxic concentrations of CO are avoided by analyzing CO or a CO marker by complementary monitoring techniques. Administering the *ex-vivo* fluid with CO via a membrane, which is liquid and solid impermeable but gas permeable, avoids contamination of the *ex-vivo* fluid by solid and dissolved residues from the reaction of the carbon monoxide releasing molecule and the triggering molecule.

The system according to the present invention, which allows the controlled administration of CO to the blood of a subject outside the body of the subject, and subsequent recirculation of the blood enriched with CO into the blood stream of the subject, is therefore useful in cardiopulmonary support, e.g. as extracorporeal life support system (ECLS), extracorporeal membrane oxygenation system (ECMO), or cardiopulmonary bypass system, or in dialysis.

### Description of the Figures

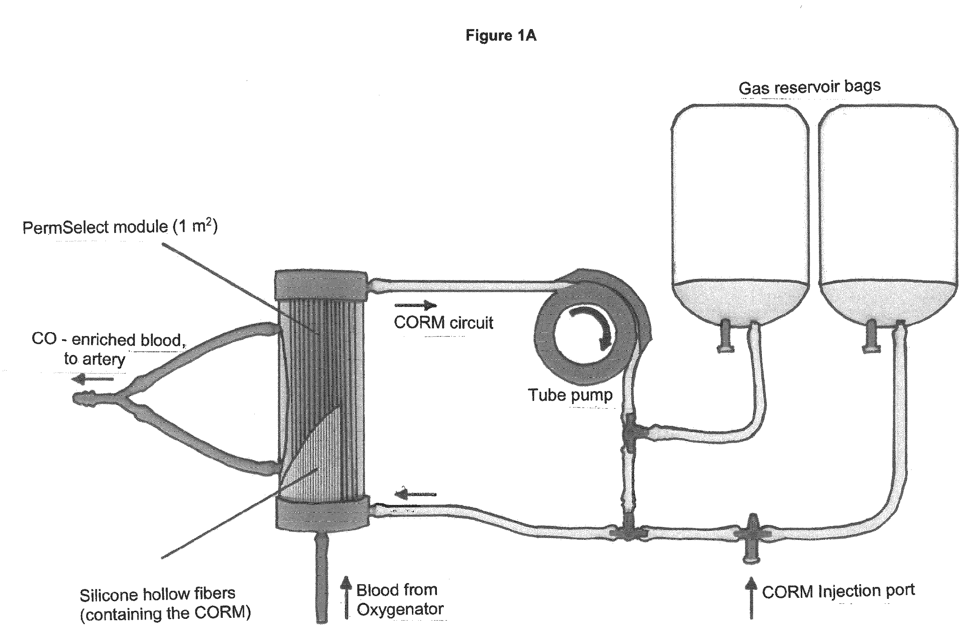

**Figure 1A** shows a schematic drawing of the membrane module being part of the extracorporeal CO releasing system (ECCORS).

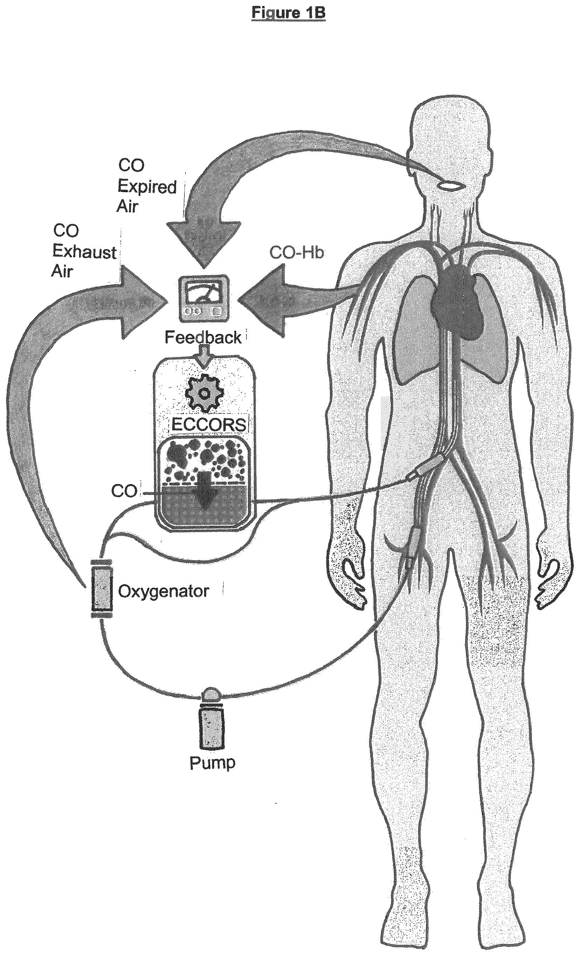

**Figure 1B** shows a schematic application of the extracorporeal CO releasing system (ECCORS) in an exemplary veno-arterial circuit of an extracorporeal life support system.

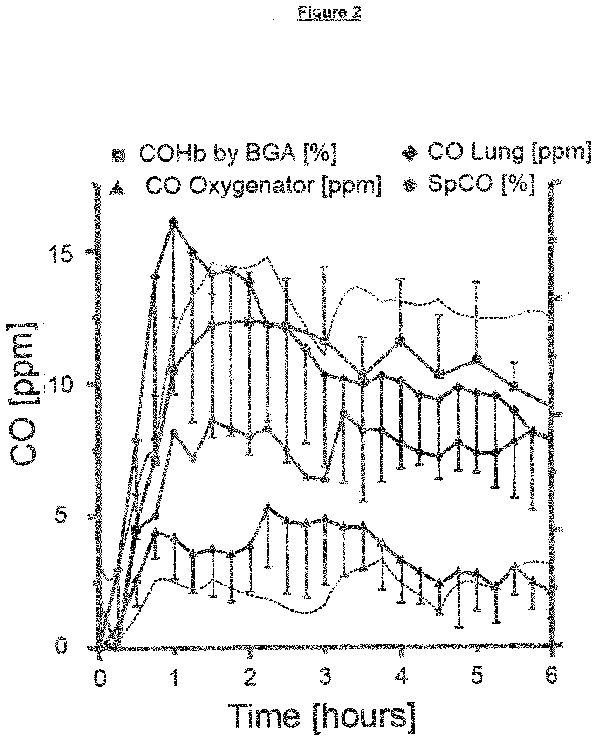

**Figure 2** shows the course of COHb (%) measured by blood gas analysis (BGA) and pulse oximetry (SpCO) as well as the concentration (ppm) of carbon monoxide exhaled from the lungs and discharged from the oxygenator. Average initiation of ECCORS treatment was at t=24.2±4.7 minutes after start of asphyxiation (t=0). N = 7; means ±SD.

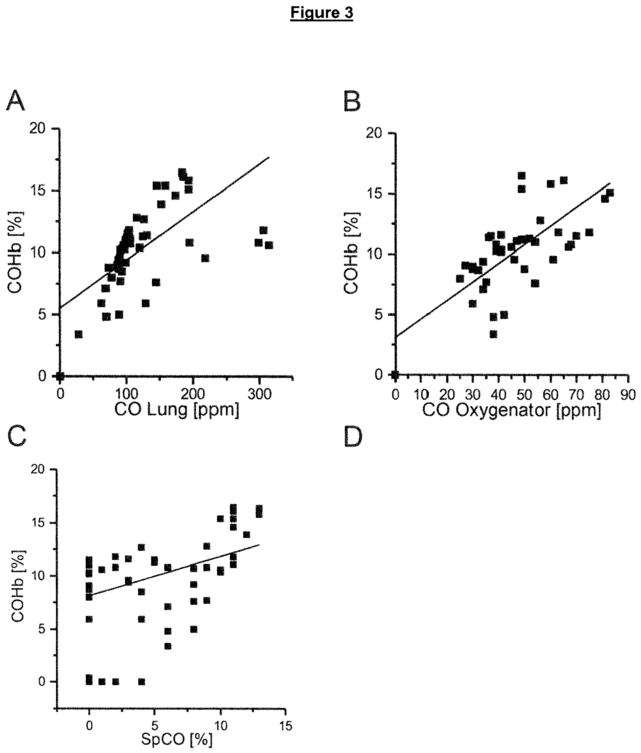

**Figure 3** shows in **(A)** a scatter plot and correlation of COHb measurement from BGA to exhaled CO from the lungs showing a correlation coefficient of R₂ = 0.43; in **(B)** a scatter plot and correlation of COHb measurement from BGA to exhausted CO from the oxygenator showing a correlation coefficient of R₂ = 0.59; in (C) a scatter plot and correlation of COHb measurement from blood gas to non-invasive SpCO measurement using pulse oximetry with a coefficient of R² = 0.37
**Figure 4** shows an example of an algorithm controlling extracorporeal carbon monoxide releasing system connected to an extracorporeal circuit system according to the invention. Systemic levels of CO are constantly assessed using four complementary monitoring techniques including COHb analysis from blood (not online), pulsoxymetric analysis of COHb, CO quantification in exhaled breath as well as exhaust of the oxygenator. These parameters are processed in an actual-target comparison approach (i) controlling the CO release rate and hence systemic CO level within the patient (including but not limited to proportional-integral-derivative controlled modulation of the kinetic between a release trigger and a CO-releasing molecule within the extracorporeal carbon monoxide releasing system) and (ii) inducing an emergency bypass of the device in case of unforeseen overdoses.

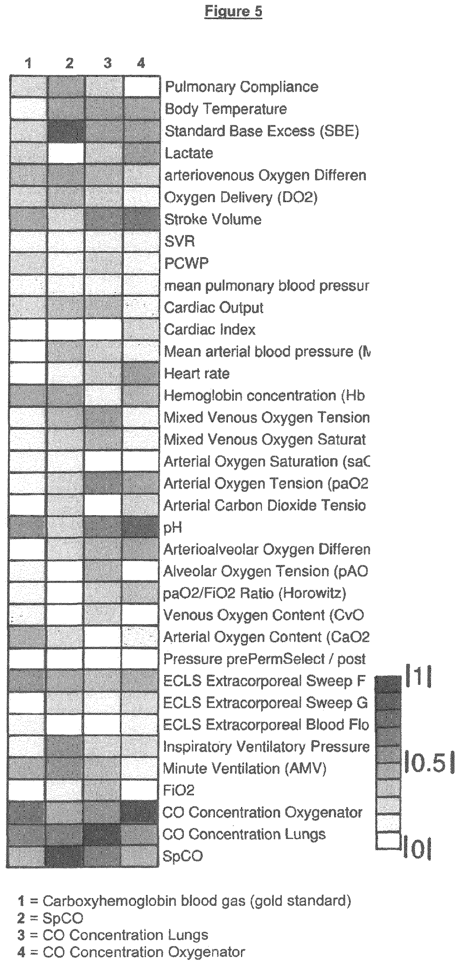

**Figure 5** shows a heat map of Pearson r correlation coefficients (absolute value) between influential parameters for COHb prediction from respiratory, acid-base and hemodynamic parameters (SpCO: COHb measured from pulse oximetry; FiO2: fraction of inspired oxygen; ECLS: extracorporeal life support; PCWP: pulmonary capillary wedge pressure; SVR: systemic vascular resistance).

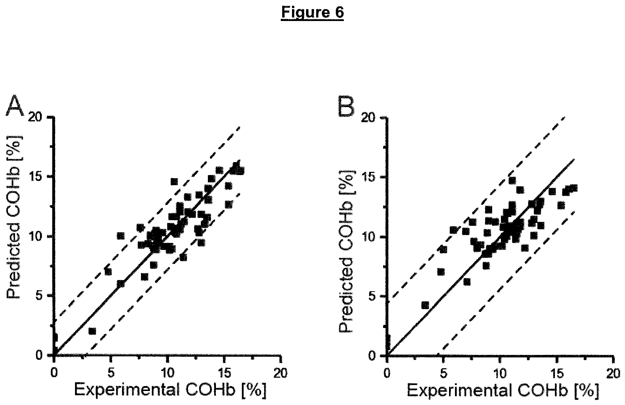

**Figure 6****.** To improve analytical precision when analyzing systemic CO levels in real-time, continuous monitoring parameters shown in **Figure 3** were processed using Random Forest regression and analyzed in randomized 5-fold cross-validations. Predictions are plotted vs. experimental COHb values: **A)** Three continuous, real-time parameters, **B)** only CO quantity in the exhaust of the oxygenator [ppm]. Solid lines indicate perfect predictions, dashed lines show the 90 % prediction interval.

### Example 1

The method and system as disclosed herein was designed for easy implementability in clinical routine. Medical-translational significance thereof is demonstrated following system development by implementing the system into a common extracorporeal circuit system setup used for preclinical testing in swine. A schematic application of a venoarterial extracorporeal circuit system comprising an extracorporeal carbon monoxide releasing system (ECCORS) for extracorporeal life support is shown in **Figure 1B****.**

After obtaining consent by Regierungspraesidium Freiburg, seven landrace-hybrid pigs (weight 52 ± 2 kg) were anesthetized (continuous infusion of propofol, fentanyl and cisatracurium), mechanically ventilated and monitored with electrocardiogram, pulse-oximetry and invasive blood pressure measurement by cannulating the carotid and pulmonary artery. Instrumentation with veno-arterial ECMO (extracorporeal membrane oxygenation) was carried out in a sterile technique: Venous access was achieved by ultrasound-guided cannulation of femoral vein (21 French HLS cannula, Maquet, Rastatt, Germany), while arterial inflow into the femoral artery was established using 17 French HLS cannula from Maquet, (Rastatt, Germany). The ECMO circuit (primary circuit) was then set-up using a customary centrifugal pump head (Revolution 5, Sorin, Rome, Italy), an oxygenator (EOS ECMO, Sorin, Rome, Italy), and 3/8" tubing for interconnection. The system was regulated using a modified console (core element: SCP-Console, Sorin, Rome, Italy).

The ECCORS was then integrated in line with the oxygenator into the primary ECMO circuit. A commercially available PDMSXA-1.0 silicone membrane module from PermSelect (AnnArbor, II) served as core functional element of the ECCORS. Silicone tube membranes within the PDMSXA module (inner diameter 190 µm; outer diameter 300 µm) separate the outer blood-carrying compartment (primary ECMO circuit) and an inner CORM-carrying compartment (secondary circuit). The outer compartment was connected to the 3/8" tubing of the primary ECMO circuit (*vide supra*)*.* The inner compartment was connected to the secondary circuit comprising a solution of 15 g FeCl₃ in 0.04 L water. The tubing of the secondary circuit was equipped with a port for CORM injection as well as two gastight bags (Reservoir bag 1 L, Sorin, Rome, Italy) for preventing overpressure in the system. The secondary circuit, was constantly circulated in ¼" tubing using a Masterflex Console Drive from Cole-Parmer (Vernon Hills, II).

**Figure 1A** shows a schematic drawing of a membrane module being part of the extracorporeal CO releasing system (ECCORS). To address safety challenges associated with pressurized CO cartridges, CO within ECCORS is generated on demand by injecting the CO releasing Molecule (CORM) Beck1 into the iron-CORM circuit, containing the trigger substance FeCl₃. After CO generation only CO (but no other constituent) permeates the gas permeable silicon hollow fibers within ECCORS and is hence delivered to the systemic circulation. The surface of the silicone hollow fibers inside the module determine the COHb concentration in steady state.

CO release was initiated by an injection of a solution of 1 g Beck1 (Mo(CO)₃(CNCH₂COOH)₃) in 0.02 L water into the secondary circuit. Further injection of Beck1 was controlled by feedback measurement to the desired target and hence based on monitoring parameters detailed in **Figure 2** and **Figure 3**, *vide infra.*

Liberated CO gas circulated in the tubing and excess gas was contained in two impermeable gas bag reservoirs connected to the circuit, enabling diffusion to the closed-loop tubing. Consequently, CO was allowed to permeate from the CORM into the blood compartment, but permeation of the non-gaseous constituents was inhibited by the membrane.

CO was quantified in the oxygenator's exhaust air, and the exhaled air using two MX6 iBrid™ (Industrial Scientific, USA) measurement devices. COHb was quantified using blood gas analysis (cobas b 123, F. Hoffmann-La Roche, Switzerland) and multi-wavelength pulse oximetry (Rad-97, Massimo, USA).

After reaching the therapeutic CO level, steady state was maintained by controlled injections of Beck1 into ECCORS under tight feedback control of online monitoring parameters including CO in exhaled air, as well as CO in the exhaust of the oxygenator **(****Figure 2****).** COHb analysis from blood samples was analyzed offline (see **Figure 2****).** This technique is the current gold standard for assessing systemic CO levels, but automatic online measurement (and hence implementation in an automatic feedback loop, *vide infra*) cannot be performed.

COHb formation was also assessed using a pulse oximeter (continuous technique), however, as this technique is primarily used by medical first responders to approximate COHb values, the data only moderately correlated with the COHb as analyzed from blood (R² = 0.37, **Figure 3C****).**

The CO level in exhaled air, as well as the CO level in the exhaust of the oxygenator was also assessed and likewise only correlates moderately to COHb as analyzed from blood samples (R² = 0.43, and 0.59, respectively, **Figures 3A** and **3B****).** Therefore, all three continuous CO-monitoring approaches alone are not appropriate to reliably distinguish between therapeutic levels (10% CO-Hb, and borderline toxicity - e.g. 16% CO-Hb) - which is one challenge to be solved by this invention.

The data showed that no toxic components of CORM, especially no transition metals can be found in the tissue or blood. Tissue was analyzed for CO effects and residual CORM constituents in the harvest organs and blood using ICP-MS analysis for molybdenum. A separation of two circuits is accomplished, such that CO is generated independently from blood flow and in a controlled fashion. By dividing two separate circuits with a solely CO-permeable, but otherwise impervious membrane, it is possible to protect organs from ischemia-reperfusion-injury by administering CO into an extracorporeal circuit without exposure of toxic CORM components to these organs.

### Example 2

A machine learning based approach was established and validated in order to increase the analytical strength of the systemic CO monitoring protocol. This approach processes the real-time CO monitoring parameters and provides a predicted CO level value for subsequent modification of the feedback loop. This protocol allows improving the analytical power of the delivery approach thereby significantly increasing the translational significance of the concept.

Method: Machine learning models were generated using Random Forest regression (Breiman L. Random Forests. Machine Learning. 2001;45(1):5-32) as implemented in the python library scikit-learn (Pedregosa F, Ga, #235, Varoquaux I, Gramfort A, Michel V, et al. Scikit-learn: Machine Learning in Python. J Mach Learn Res. 2011;12:2825-30). The number of estimators was set to 1000. The predictive power of the models was assessed in randomized 5-fold cross-validations and in leave-one-animal-out cross-validations.

Results: During *in-vivo* studies, multiple parameters (see **Figure 5****)** were plotted and analyzed, accordingly. In correlation studies, the factors CO *Lungs, CO Oxygenator,* and *SpCO* (carboxyhemoglobin measurement from pulse oximetry) were identified as most influential for COHb prediction with R₂ values of 0.43, 0.59, and 0.37, respectively (see **Figures 3** **A, B, C).**

In an effort to increase accuracy and precision of the analytical setup, we used these parameters to generate a multifactorial COHb prediction model. As baseline, the predictive power of a multiple linear regression (MLR) approach was assessed in a randomized 5-fold cross-validation (CV) and a leave-one-animal-out CV, respectively. The three-parameter MLR model predicted COHb values with a mean absolute error (MAE) of 2.36 % COHb and a Q² (R² of CV prediction results) of 0.45, suggesting moderate prediction quality (see Table 1). The leave-one-animal-out CV indicates poorer performance of the MLR model with an MAE of 2.82 % and a Q² of 0.10. To improve the quality of COHb predictions, non-linear machine learning models were built using Random Forest regression (RF). Using RF, the COHb prediction power increased to an MAE of 1.35 % at a Q² of 0.79 in the randomized 5-fold CV and an MAE of 1.41 % at a Q² of 0.72 in the leave-one-animal-out CV. The randomized 5-fold CV indicates a 90%-prediction interval of 2.80 % COHb (Figure 6A). Random forest regression was repeated with CO Oxygenator as the only input parameter resulting in a prediction model with an MAE of 2.16 % at a Q² of 0.52 in a randomized 5-fold CV **(****Figure 6B****)** and an MAE of 1.99 % at a Q² of 0.46 in a leave-one-animal-out CV.

## Claims

1. A method for combined administration of carbon monoxide (CO) to an *ex-vivo* fluid and monitoring of the carbon monoxide administration, said method comprising:
(i) generating CO by reacting a CO releasing molecule (CORM) with a release triggering molecule,
(ii) administering CO to an *ex-vivo* fluid by contacting the *ex-vivo* fluid with the CO generated in step (i) via a gas-permeable membrane,
(iii) analyzing carbon monoxide and/or a carbon monoxide marker after administering in step (ii) CO to the *ex-vivo* fluid by complementary monitoring techniques,
(iv) adjusting the CO administration based on the analysis of the carbon monoxide or the carbon monoxide marker carried out in step (iii), if necessary.

2. The method according to claim 1, wherein the *ex-vivo* fluid is blood of a subject or perfusion liquid, and preferably blood of a subject.

3. The method according to claim 1 or 2, wherein the carbon monoxide marker is carboxyhemoglobin (COHb).

4. The method according to any of claims 1 to 3, wherein the complementary monitoring techniques include at least two of: COHb measurement in a blood sample of a subject, measuring the CO concentration in the exhaled breath of a subject, and measuring the CO concentration in the exhaust air of an oxygenator, the oxygenator being integrated in a cardiopulmonary support system connected with the blood cycle of a subject.

5. The method according to any of claims 1 to 4, wherein the complementary monitoring techniques applied in step (iii) is supported by machine learning-based CO level prediction, preferably by using Random Forest regression.

6. An extracorporeal circuit system for use in a method according to any of claims 1 to 5, said extracorporeal circuit system comprising:
an extracorporeal carbon monoxide releasing system (ECCORS) comprising a membrane module with an outer and an inner compartment,
wherein the outer compartment is connected to a primary circuit carrying blood of a subject, and the inner compartment is connected to a secondary circuit wherein CO is generated by reacting a CO releasing molecule with a release triggering molecule; and wherein the two compartments in the membrane module are separated from each other by a gas-permeable membrane that allows CO permeation generated in the secondary circuit from the inner compartment into the outer compartment, thereby administering CO to the *ex-vivo* fluid,
wherein the primary circuit is connected with the blood cycle of a subject.

7. The extracorporeal circuit system according to claim 6, wherein the inner and the outer compartment of the membrane module are separated by tube membranes.

8. The extracorporeal circuit system according to claim 6 or 7, wherein the primary circuit further comprises an oxygenator and a pump.

9. The method according to any of claims 1 to 5 and the extracorporeal circuit system for use according to any of claims 6 to 8, wherein the gas-permeable membrane is a silicone membrane or a PTFE (polytetrafluoroethylene) membrane.

10. The method according to any of claims 1 to 5 and 9 and the extracorporeal circuit system for use according to any of claims 6 to 9, wherein the CORM is a metal carbonyl compound.

11. The method according to any of claims 1 to 5, 9 and 10 and the extracorporeal circuit system for use according to any of claims 6 to 10, wherein the CORM is a metal carbonyl compound selected from the group consisting of Mo(CO)₃(CNCH₂COOH)₃ ("Beck1"), Mo(CO)₃(CNCH₂CONaO)₃ ("Beck1-Na"), CORM-ALF794, CORM-1, CORM-2, CORM-3, and CORM-401.

12. The method according to any of claims 1 to 5 and 9 to 11 and the extracorporeal circuit system for use according to any of claims 6 to 11, wherein the release triggering molecule is selected from the group consisting of a sulfur containing compound, a nitrogen containing compound, and an oxidizing compound.

13. The method according to any of claims 1 to 5 and 9 to 12 and the extracorporeal circuit system for use according to any of claims 6 to 12, wherein the release triggering molecule is an oxidizing compound selected from the group consisting of iron(III)chloride (FeCl₃), potassium permanganate (KMnO₄), cer(IV)sulfate (Ce(SO₄)₂), potassium dichromate K₂Cr₂O₇, gold(III)chloride (AuCl₃), and silver nitrate (AgNO₃).

14. The method according to any of claims 1 to 5 and 9 to 13 and the extracorporeal circuit system for use according to any of claims 6 to 13, wherein the CORM is added to an aqueous solution of the release triggering molecule or the release triggering molecule is added to an aqueous solution of the CORM.

15. The extracorporeal circuit system for use according to claims 5 to 14, wherein the extracorporeal circuit system is part of a cardiopulmonary support system or a dialysis system.
